# EUROPEAN PATENT APPLICATION

(11) **EP 2 405 267 A1**
(43) Date of publication of application: **11.01.2012**
(21) Application number: 10748721.7
(22) Date of filing: 01.03.2010
(51) Int. Cl.: G01N 33/543, G01N 33/53

(54) **SURFACE-MODIFIED BASE PLATE, BIOCHIP AND PROCESS FOR PRODUCING THE BIOCHIP**

(30) Priority: 02.03.2009 JP 2009047518
(71) Applicant: Shiseido Co., Ltd., Tokyo 104-8010 (JP)
(72) Inventor: MAENO, Katsuyuki, Yokohama-shi Kanagawa 224-8558 (JP)
(74) Representative: TER MEER - STEINMEISTER & PARTNER GbR
(86) International application number: PCT/JP2010/053278
(87) International publication number: WO 2010/101126

(57) **Abstract**

A surface modified substrate is produced by a method including reacting a substrate having a reactive functional group on a surface thereof with a functional group being reactive to the reactive functional group, and reacting the substrate with a compound represented by the general formula, 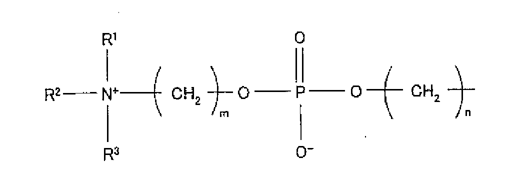 wherein R¹, R² and R³ are each independently an alkyl group with a carbon number of 1 or more and 6 or less, m is an integer from 2 to 6, and n is 1 or 2.

## Description

### TECHNICAL FIELD

The present invention relates to a surface modified substrate, a biotip, a method for producing thereof, a biosensor and an analytical method.

### BACKGROUND ART

Conventionally, a polymer having a phosphorylcholine group is known as a biocompatible polymer and various kinds of resin material coated by such a polymer are also known as a biocompatible material.

A Patent Document 1 discloses a material for medical use. The material has a coating layer composed of a copolymer of a monomer having a phosphorylcholine-like group as a side chain and a monomer having a group capable of bonding to heparin or a heparin derivative.

Moreover, a Patent Document 2 discloses a separation material having a phosphorylcholine-like group at least on the surface. The ratio (P/C) of the quantity of phosphorous element P originating from the phosphorylcholine-like group to the quantity of carbon element C is 0.002-0.3 based on the X-ray photoelectron spectroscopic analysis of the surface.

However, a material coated by a polymer having a phosphorylcholine group has a problem with easily adsorbing biological materials, because a phosphorylcholine group cannot be introduced at high density.
[Patent Document 1] Japanese Patent Application Publication No.2000-279512
[Patent Document 2] Japanese Patent Application Publication No.2002-098676

### DISCLOSURE OF THE INVENTION

### PROBLEMS TO BE SOLVED BY THE INVENTION

While taking the problem above mentioned in conventional arts into consideration, the present invention is intended to provide a surface modified substrate and a biotip which may reduce the adsorption of a biological material, a method for producing the tip, a biosensor having the tip and an analytical method by using the biotip.

### MEANS FOR SOLVING PROBLEMS

The invention as recited in claim 1 is a surface modified substrate produced by a method, the method including: reacting a substrate having a reactive functional group on a surface thereof with a functional group being reactive to the reactive functional group; and reacting the substrate with a compound represented by the general formula 1, 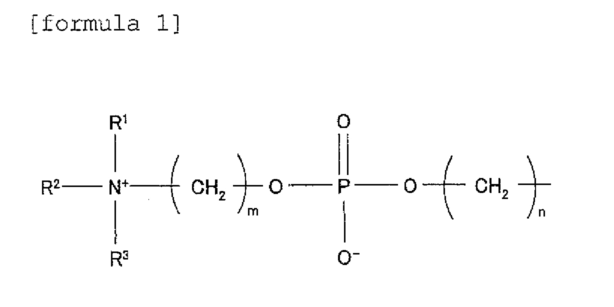 wherein, R¹ R² and R³ are each independently an alkyl group with a carbon number of 1 or more and 6 or less, m is an integer from 2 to 6, and n is 1 or 2.

The invention as recited in claim 2 is the surface modified substrate as claimed in claim 1, wherein the reactive functional group is one or more groups selected from a group consisting of an amino group, a hydroxy group, an aldehyde group, a carboxyl group, a functional group capable of being hydrolyzed to form a silanol group, and a silanol group.

The invention as recited in claim 3 is a biotip produced by a method comprising reacting the surface modified substrate according to claim 1 or 2 with a ligand having a functional group being reactive to the reactive functional group.

The invention as recited in claim 4 is a method for producing a biotip comprising steps of: reacting a substrate having a reactive functional group on the surface with a ligand having a functional group being reactive to the reactive functional group; and reacting the substrate reacted with the ligand with a compound represented by the general formula 2, 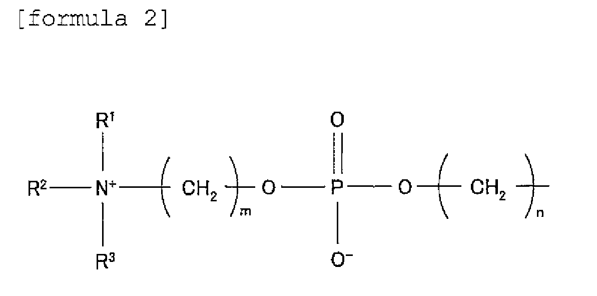 wherein, R¹, R² and R³ are each independently an alkyl group with a carbon number of 1 or more and 6 or less, m is an integer from 2 to 6, and n is 1 or 2.

The invention as recited in claim 5 is a biotip produced by the method for producing a biotip according to claim 4.

The invention as recited in claim 6 is a biosensor comprising the biotip according to claim 3 or 5.

The invention as recited in claim 7 is an analytical method for a material being selectively bound to a ligand, comprising using the biosensor according to claim 6.

### EFFECT OF THE INVENTION

According to the present invention, it is possible to provide a surface modified substrate and a biotip which may reduce the adsorption of a biological material, a method for producing the tip, a biosensor having the tip and an analytical method by using the biotip.

### BRIEF DESCRIPTION OF DRAWINGS

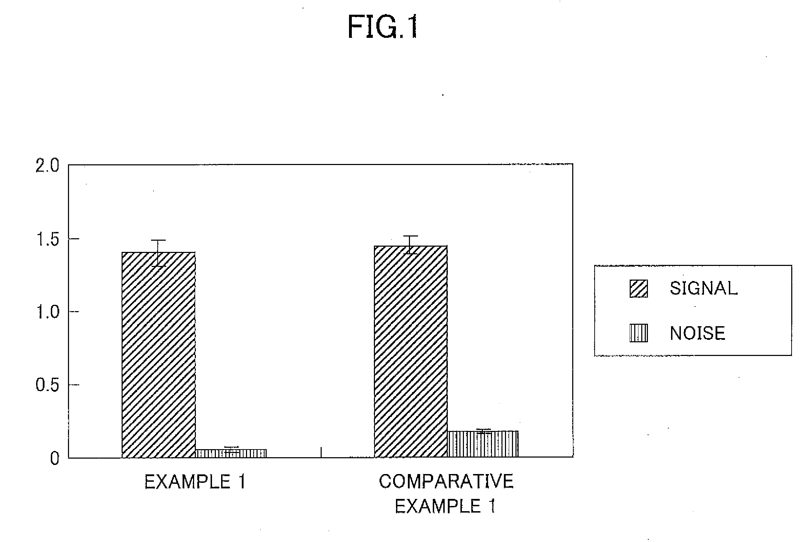

FIG.1 shows evaluation results of the ratio S/N according to Example 1 and Comparative Example 1.

### BEST MODE FOR CARRYING OUT THE INVENTION

A mode for carrying out the present invention will be described hereinafter together with the drawing.

A surface modified substrate of the present invention is produced by reacting a substrate having a reactive functional group on the surface thereof with a functional group being reactive to the reactive functional group, and reacting the substrate with a compound represented by the general formula 3, 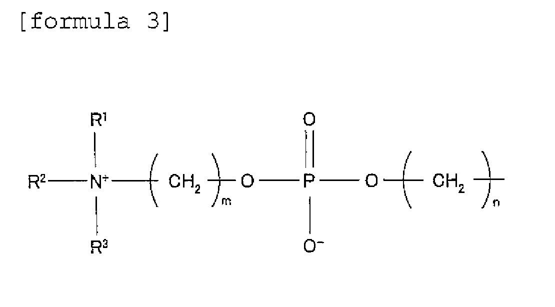 wherein, R¹, R² and R³ are independently an alkyl group with a carbon number of 1 or more and 6 or less, m is an integer from 2 to 6, and n is 1 or 2. By using this method a phosphorylcholine-like group may be introduced on the substrate surface at high density. Accordingly, a surface modified substrate which may reduce the adsorption of biological material may be obtained.

In the present invention, a reactive functional group on the substrate surface includes, but is not limited to, an amino group, a hydroxyl group, an aldehyde group, a carboxyl group, a functional group capable of being hydrolyzed to produce a silanol group, a silanol group, any combination of these groups and the like. A functional group capable of being hydrolyzed to produce a silanol group includes, but is not limited to, a hydrosilyl group, an alkoxysilyl group, a halosilyl group, an acyloxysilyl group, an aminosilyl group and the like. An alkoxysilyl group having carbon number 1-6 or a hydrosilyl group is preferable from stability, reactivity or the like points of view.

A material for the substrate having such a reactive functional group may be either an organic material or an inorganic material.

The organic material includes, but is not particularly limited to, a homopolymer or copolymer obtained by a polymerization of a monomer such as a styrene, a glycidyl methacrylate, a (meth) acrylic acid, a N-alkylacrylamide, an alkyl (meth)acrylate, an amino alkyl (meth)acrylate, a hydroxyalkyl (meth)acrylate or the like. Particularly an acrylic
acid-N-isopropylacrylamide-methylenebisacrylamide copolymer, a 2-hydroxyethyl
methacrylate-styrene-divinylbenzene copolymer, a 2-aminoethylmethacrylate-N-isopropylacrylamide-methylene bisacrylamide copolymer or the like is preferable. Other organic material includes agarose or sepharose and the like.

The inorganic material includes, but is not particularly limited to, talc, kaolin, mica, sericite, muscovite, phlogopite, synthetic mica, red mica, biotite, permiculite, magnesium carbonate, calcium carbonate, aluminum silicate, barium silicate, calcium silicate, magnesium silicate, strontium silicate, metal salt of tungstic acid, magnesium, silica, zeolite, barium sulfate, Fired calcium sulfate (Gypsum Fired), calcium phosphate, fluorine apatite, hydroxyapatite, ceramic powder, metal soap (for example, zinc myristate, calcium palmitate, aluminium stearate), boron nitride, cerium oxide and the like. In particular silica, titanium oxide, zinc oxide, alumina, iron oxide, talc, mica, sericite or the like is preferable.

A substrate having a reactive functional group may be a substrate of which surface is treated to introduce a reactive functional group.

A method for introducing an amino group on the substrate surface includes a plasma treatment, a reaction with a surface treatment agent or a silicone vapor treatment.

The plasma treatment introduces an amino group on a particle surface by applying a low temperature plasma under nitrogen atmosphere (see for example, Surface and Coatings Technology 116-119(1999) 802-807, Colloids and Surfaces A: Physicochem. Eng. Aspects 195(2001) 81-95, Macromol. Chem. Phys. 200, 989-996(1999)). Specifically after a substrate is inserted into a reaction chamber and the chamber is evacuated by a vacuum pump, nitrogen gas is introduced into the chamber and a glow discharging is applied.

In the method of reaction with a surface treatment agent, an amino group is introduced on the substrate surface having silanol group, alkoxysilyl group and the like by using a surface treatment agent such as alkoxysilane, chlorosilane, or silazane having an amino group. Specifically, such substrate is immersed in a mixture of water/2-propanol, and
3-aminopropyltrimethoxysilane is added into the mixture. Then, the mixture is heated up to 100°C, and kept for 6 hours to complete the reaction. After cooling down to the room temperature, the substrate is washed with methanol and dried.

A substrate material includes an organic material such as 3-trimethoxysilylpropyl methacrylate-methyl methacrylate-divinylbenzene copolymer; and an inorganic material such as silica, glass, alumina, talc, clay, mica, asbestos, titanium oxide, zinc oxide, iron oxide and the like.

In the silicone vapor treatment, an amino group is introduced on the substrate by introducing hydrosilyl group on the surface by using
1,3,5,7-tetramethylcyclotetrasiloxane followed by reaction with amino group containing monomer (See, for example, Japanese Patent publications of examined applications 1989-54379, 1989-54380, 1989-54381). Specifically, a substrate and 1,3,5,7-tetramethylcyclotetrasiloxane are set in a desiccator together, and the desiccator is evacuated by using an aspirator. After 16 hours of reaction at 80 °C, the substrate is taken out and dried at 120°C. The substrate is then immersed in ethanol. An allylamine is added to the solution, and then an ethanol solution of chloroplatinic acid is added. The solution is kept at 60°C for 2 hours under stirring. After the reaction is completed, the substrate is washed with ethanol and dried under vacuum.

A material for a substrate includes an organic material, such as styrene-divinylbenzene copolymer and the like, and an inorganic material, such as mica, talc, kaolin, alumina, titanium oxide, zinc oxide, iron oxide and the like.

An amino group containing monomer is not limited to an allylamine, but may be a vinyl monomer or an acryl monomer containing an amino group. Further the amino group may be protected with a butoxycarbonyl group or a benzyloxycarbonyl group and the like. Furthermore, instead of an amino group containing monomer, a monomer having a functional group such as an epoxy group, capable of reacting with, for example, a diamine may be used to introduce an amino group on the substrate.

A method for introducing an aldehyde group on the substrate surface includes, for example, a reaction for a glutaraldehyde with an amino group on the substrate surface.

A method for introducing a carboxyl group on the substrate surface includes, for example, a reaction with a surface treating agent or silicone vapor treating and the like.

In a method of reacting with a surface treating agent, a carboxyl group is introduced on the substrate surface having a silanol group, an alkoxysilyl group and the like by using a surface treating agent such as an alkoxysilane, a chlorosilane, a silazane or the like having a carboxyl group. Specifically, a silane coupling agent having a carboxyl group is synthesized as follows. Triethoxysilylpropylsuccinic anhydride is dissolved in N,N-dimethylformamide. To the solution are added distilled water and 4-dimethylaminopyridine, and the mixture is stirred for 16 hours to produce the silane coupling agent. The substrate is immersed in the water/2-propanol mixture, and the coupling agent having a carboxyl group is added. The mixture is heated to 100°C for 6 hours to complete the reaction. After cooling to the room temperature, the substrate is washed with methanol and dried.

A material for the substrate includes an organic material such as 3-trimethoxysilylpropyl methacrylate-methyl methacrylate-divinylbenzene copolymer, and an inorganic material such as silica, glass, alumina, talc, clay, mica, asbestos, titanium oxide, zinc oxide, iron oxide and the like.

In the silicone vapor treatment, a carboxyl group is introduced on the substrate by introducing hydrosilyl group on the surface by using 1,3,5, 7-tetramethylcyclotetrasiloxane followed by reaction with a carboxyl group containing monomer (See, for example, Japanese Patent publications of examined applications 1989-54379, 1989-54380, 1989-54381). Specifically, a substrate and 1,3,5,7-tetramethylcyclotetrasiloxane are set in a desiccator together, and the desiccator is evacuated by using an aspirator. After 16 hours of reaction at 80 °C, the substrate is taken out and dried at 120°C. The substrate is then immersed in ethanol. An allylcarboxylic acid is added to the solution, and then an ethanol solution of chloroplatinic acid is added. The solution is kept at 60°C for 2 hours under stirring. After the reaction is completed, the substrate is washed with ethanol and dried under vacuum.

A material for the substrate includes an organic material such as styrene-divinylbenzene copolymer and the like, and an inorganic material such as mica, talc, kaolin, alumina, titanium oxide, zinc oxide, iron oxide and the like.

A monomer having a carboxyl group is not limited to allylcarboxylic acid, and may be a vinyl monomer or acryl monomer having a carboxyl group.

A method for introducing a functional group capable of being hydrolyzed to produce silanol and/or silanol group on the substrate surface includes, for example, to coat the surface with a coating liquid containing a polymer having a functional group capable of being hydrolyzed to produce an silanol group and an alkoxylsilane.

By applying the coating liquid containing a polymer capable of being hydrolyzed and an alkoxysilane on the substrate surface, the polymer and the alkoxysilane are hydrolyzed to produce silanol group. Further dehydration condensation between such silanols causes crosslinking of the polymers to form a cross linked polymer layer containing silanol group. Specifically, after coating the coating liquid on the material, water, acid or alkaline may be applied on the material, or heating may be applied on the material. Alternatively, the coating liquid may be applied on the material after coating water, acid or alkaline on the material. Water, acid or alkaline may be mixed with the coating liquid. In this case, it is preferable to prepare the coating liquid just during the coating time, since hydrolysis reaction is taking place in the coating liquid. When water, acid or alkaline is used, heating may be applied, although the reaction usually takes place sufficiently at room temperature. Without using water, acid or alkaline, the reaction can usually take place gently with only atmospheric moisture.

Any acid or alkaline may be used for hydrolysis without any limitation if it can hydrolyze. Any mixture of more than two kinds of acid or alkaline may be used. Also an acid or an alkaline for hydrolysis reaction may be used in aqueous solution.

A coating liquid may be a solution or a dispersion of a polymer capable of being hydrolyzed and alkoxysilane in an organic solvent. The organic solvent includes, but is not particularly limited to, an aliphatic hydrocarbon, an aromatic hydrocarbon, a chlorinated hydrocarbon, an ether type solvent, an alcohol type solvent of an aliphatic alcohol having carbon number 1-4 and 1-4 hydroxyl group, a cellsolve type solvent such as an ethylcellsolve, a butylcellsolve and the like, dioxane, methyl acetate, dimethylformamide or the like. A mixture of 2 or more solvents may be used.

The concentration of a polymer capable of being hydrolyzed is preferably 0.001-20 weight% and more preferably 0.1-5 weight%. When the concentration of the polymer is less than 0.001 weight%, sufficient effect may not be achieved by one time treatment. When the concentration of the polymer is more than 20 weight%, coating properties or the like may be reduced.

The weight ratio of a polymer capable of being hydrolyzed to alkoxysilane is preferably 0.01%-20% and more preferably 0.2%-5%. When the weight ratio is less than 0.01%, cross linked polymer strength may be insufficient. When the weight ratio is more than 20%, the amount of silanol group introduced into the crosslinked polymer may be insufficient.

A coating method includes, but is not particularly limited to, a dipping coating, a spray coating, a spin casting or the like.

A material for a substrate includes an organic material such as PP (polypropylene), polycarbonate, PET (polyethyleneterephthalate), PEEK, fluorinated resin, polystyrene, vinylchloride and the like, and an inorganic material such as gold, titanium, aluminum, iron, copper, stainless, alumina, titanium oxide, zinc oxide and the like.

A polymer capable of being hydrolyzed is not particularly limited to, but may be a polymer which may form a silanol group on hydrolysis. A homopolymer or copolymer (polymer (A) hereinafter) obtained by a polymerization of a monomer (A-1) represented in a general formula 4 may be used. 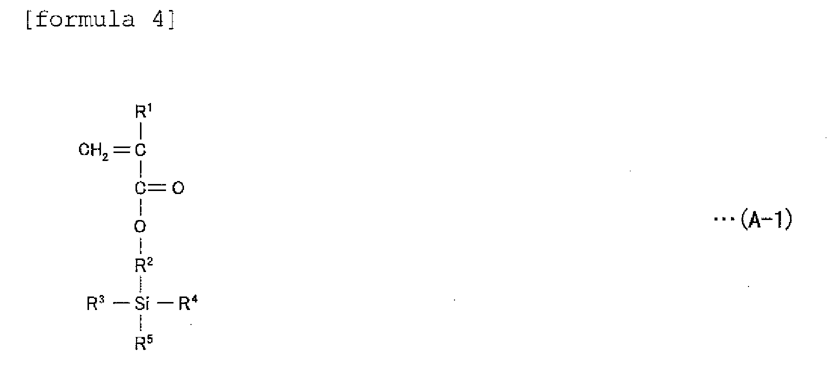 Wherein, R¹ is hydrogen or methyl group; R² is an alkylene group having 1-6 carbon atom(s), preferably propylene; R³, R⁴ and R⁵ are each independently an alkoxy group having 1-6 carbon atom(s), preferably methoxy or ethoxy group.

In the polymerization, two or more monomers (A-1) may be used.

In the polymerization for the polymer (A), a monomer (A-2) represented in a general formula 5 may be used together to produce a copolymer. 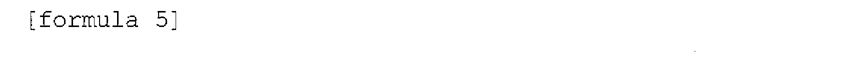 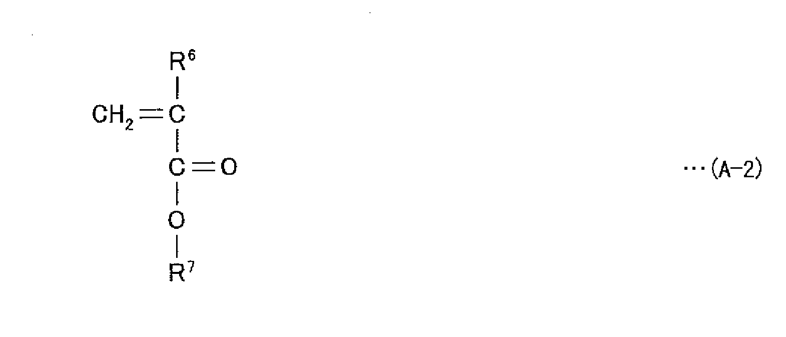 Wherein, R⁶ is a hydrogen atom or a methyl group; R⁷ is a linear, branched or cyclic hydrocarbon having 1-18 carbon atom(s), preferably alkyl group having 1-6 carbon atom(s), particularly preferably a methyl group.

In the copolymerization, two or more monomers (A-2) may be used.

In the polymerization for the polymer (A), a monomer (A-3) represented in a general formula 6 may be used together to produce a copolymer. 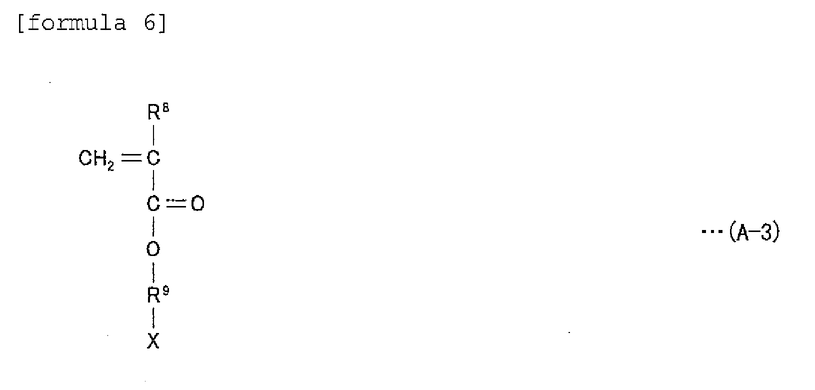 Wherein, R⁸ is a hydrogen or a methyl group; R⁹ is an alkylene group having 1-6 carbon atom(s), preferably an ethylene, a propylene or a 2-hydroxypropylene; X is a functional group (X-1) represented by a general formula 7, 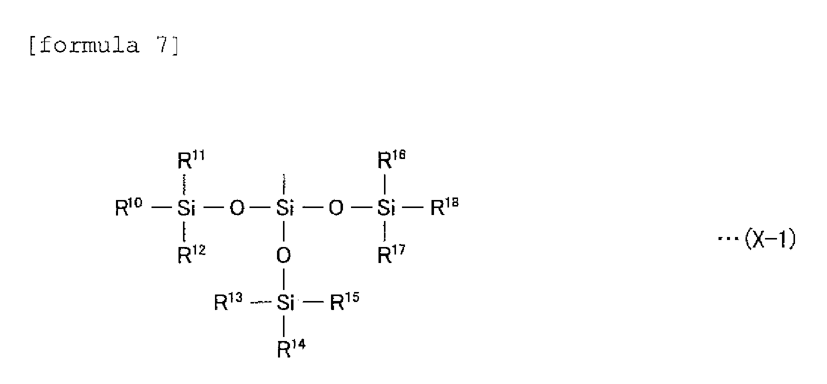 wherein, R¹⁰, R¹¹, R¹², R¹³, R¹⁴, R¹⁵, R¹⁶, R¹⁷ and R¹⁸ are each independently, a linear or branched alkyl group having 1-6 carbon atom(s), preferably a methyl group, a functional group (X-2) represented by a general formula 8, 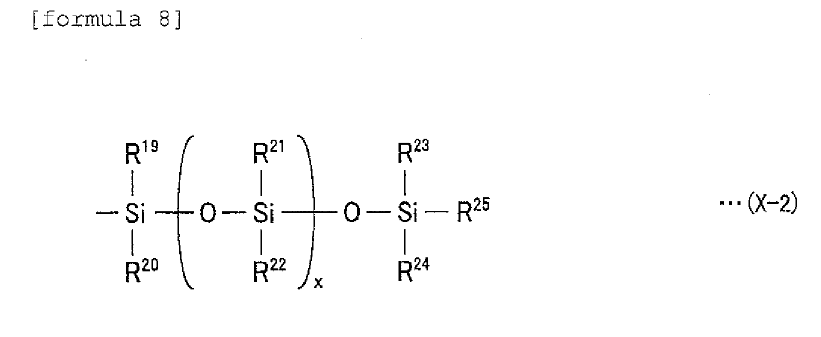 wherein, R¹⁹, R²⁰, R²¹, R²², R²³ and R²⁴ are each independently a linear or branched alkyl group having 1-6 carbon atom(s), preferably a methyl group; R²⁵ is a linear or branched alkyl group having 1-6 carbon atom(s), preferably a butyl group; x is positive integer,
or
a functional group (X-3) represented by a general formula 9, 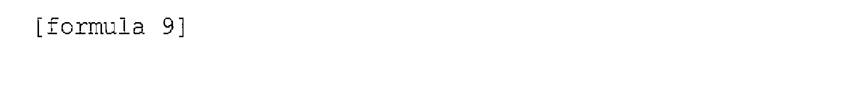 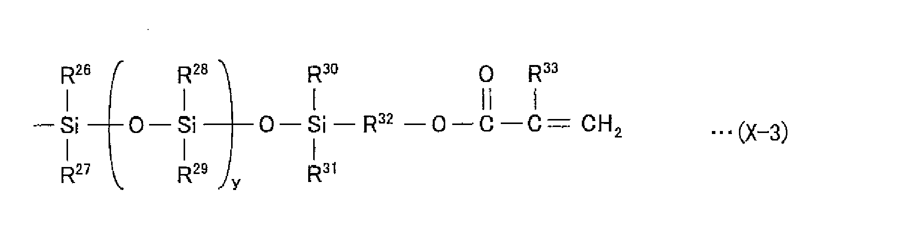 wherein, R²⁶, R²⁷, R²⁸, R²⁹, R³⁰ and R³¹ are each independently a linear or branched alkyl group having 1-6 carbon atom(s), preferably a methyl group; R³² is an alkylene group having 1-6 carbon atom(s), preferably an ethylene group, a
propylene group or a 2-hydroxypropylene group; R³³ is a hydrogen or a methyl group; y is positive integer. When X is (X-2) or (X-3), the molecular weight of monomer (A-3) is preferably 1000-100000 and particularly preferably 2000-20000.

In the copolymerization, two or more monomers (A-3) may be used.

In the polymerization for the polymer (A), a monomer (A-4) represented in a general formula 10 may be used together to produce a copolymer. 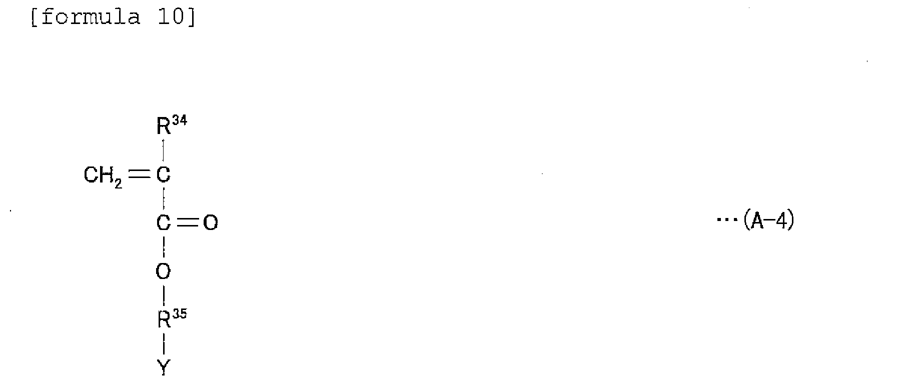 Wherein, R³⁴ is a hydrogen or a methyl group; R³⁵ is an alkylene group having 1-6 carbon atom(s), preferably an ethylene or a propylene; Y is a functional group (Y-1) represented by a general formula 11, 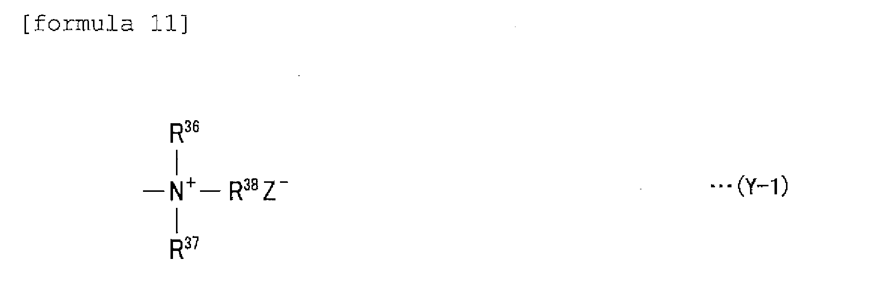 wherein, R³⁶, R³⁷ and R³⁸ are each independently, an alkyl group having 1-6 carbon atom(s), preferably a methyl group; Z⁻ is halide ion or a conjugate ion of an organic or an inorganic acid,
or
a functional group (Y-2) represented by a general formula 12, 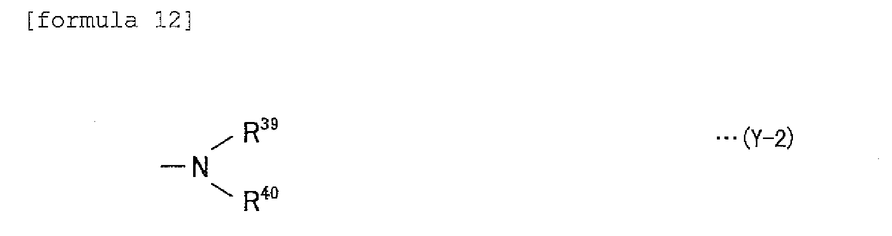 wherein, R³⁹ and R⁴⁰ are each independently an alkyl group having 1-6 carbon atom(s), preferably a methyl group. In the copolymerization, two or more monomers (A-4) may be used.

In other words, for synthesizing polymer (A), monomer (A-1) may be copolymerized with at least one monomer of monomer (A-2), monomer (A-3) or monomer (A-4).

The amount of monomer (A-1) in all monomers for polymer (A) synthesis is preferably 40-85 weight%. When the amount of monomer (A-1) is less than 40 weight%, the crosslink density may be too low to maintain the hydrophilic property sufficiently. When the amount of monomer (A-1) is more than 85 weight%, the uniformity of the crosslinked polymer layer may be reduced.

The amount of monomer (A-2) in all monomers for polymer (A) synthesis is preferably 1 weight% or more, and more preferably 10 weight% or more. When the amount of monomer (A-2) is less than 1 weight%, a water resistance of the crosslinked polymer layer may be reduced. Furthermore, the amount of monomer (A-2) in all monomers for polymer (A) synthesis is preferably 75 weight% or less, and more preferably 60 weight% or less. When the amount of monomer (A-2) is more than 75%, the polymer (A) may be insoluble in an alcoholic solvent.

The amount of monomer (A-3) in all monomers for polymer (A) synthesis is preferably 1 weight% or more, and more preferably 5 weight% or more. When the amount of monomer (A-3) is less than 1%, a water resistance of the crosslinked polymer layer may be reduced. Furthermore, the amount of monomer (A-3) in all monomers for polymer (A) synthesis is preferably 70 weight% or less, and more preferably 60 weight% or less. When the amount is more than 70%, the polymer (A) may be insoluble in an alcoholic solvent.

The weight ratio of monomer (A-4) to total weight of all monomers (A-1), (A-2) and (A-3) is preferably 0.01-1 and more preferably 0.05-0.5. When the ratio is less than 0.01, the flexibility of the crosslinked polymer layer may be reduced. When the ratio is more than 1, a water resistance of the crosslinked polymer layer may be reduced.

The number average molecular weight of polymer (A) is not particularly limited to, but more than that of an oligomer. The number average molecular weight of polymer (A) is preferably 2000-150000. When the number average molecular weight is less than 2000, it may take a long time to form a crosslinked polymer layer. When the number average molecular weight is higher than 150000, the viscosity of the coating liquid may be too high, which impairs the coating properties or workability.

A specific method for producing polymer (A) is disclosed in Japanese Patent Publication 1999-302129.

A homopolymer or copolymer (hereinafter polymer (B)) having a unit (B-1) represented in a general formula 13 may be used as a polymer capable of being hydrolyzed. 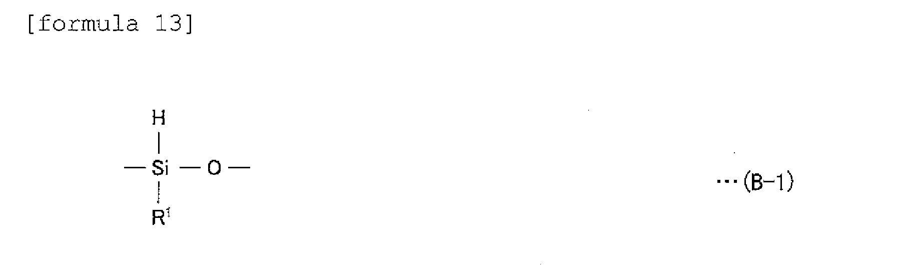 Wherein, R¹ is an alkyl group having 1-22 carbon atom(s) or a phenyl group, preferably a methyl group.

Polymer (B) may comprise two or more units (B-1).

Further, polymer (B) may comprise a unit (B-2) represented in a general formula 14. 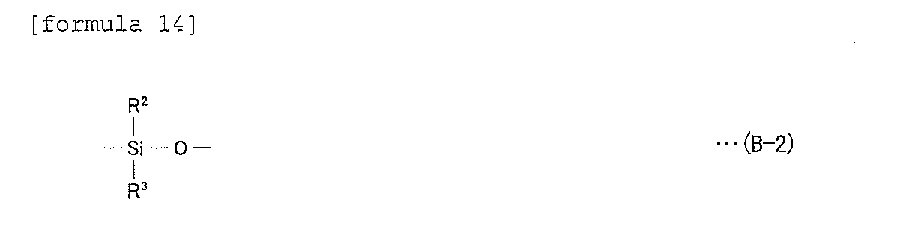 Wherein, R² and R³ is each independently an alkyl group having 1-22 carbon atom(s) or a phenyl group, preferably a methyl group.

Polymer (B) may comprise two or more units (B-2).

The amount of monomer (B-1) in all monomers for polymer (B) synthesis is preferably 1-90 weight%. When the amount of unit (B-1) is less than 1 weight%, the crosslink density may be reduced to maintain the hydrophilic property sufficiently. When the amount of unit (B-1) is more than 90 weight%, the uniformity of crosslinked polymer layer may be reduced.

The amount of monomer (B-2) in all monomers for polymer (B) synthesis is preferably 10-99 weight%. When the amount of unit (B-2) is less than 10 weight%, the uniformity of crosslinked polymer layer may be reduced. When the amount of unit (B-2) is more than 99 weight%, the crosslink density may be reduced to maintain the hydrophilic property sufficiently.

The number average molecular weight of polymer (B) is not particularly limited to, but more than that of an oligomer. The number average molecular weight of polymer (B) is preferably 2000-500000. When the number average molecular weight is less than 2000, it may take a long time to form crosslinked polymer layer. When the number average molecular weight is higher than 500000, the viscosity of the coating liquid may be too high, which impairs the coating properties or workability.

A polymer (A) and polymer (B) may be used together as a polymer capable of being hydrolyzed. Also a polymer capable of being hydrolyzed and a polymer incapable of being hydrolyzed may be used together. A polymer incapable of being hydrolyzed includes, but is not particularly limited to, for example polymer (A) or polymer (B) having no functional group which is hydrolyzed to form a silanol group.

A method for introducing a silanol group on the substrate surface includes, for example, to coat a coating liquid containing a silicone resin to form a layer containing silicone resin having a silanol group.

The layer containing silicone resin having a silanol group preferably has a water contact angle of 3-8 degrees. When the water contact angle is less than 3 degrees, it may be difficult to form the layer. When the water contact angle is more than 8 degrees, it may be difficult to introduce a phosphorylcholine-like group on the surface at high density.

A silicone resin contained in a coating liquid includes, but is not particularly limited to, for example a resin obtained by a condensation after hydrolysis of an alkoxysilane represented in a general formula:

(R¹O)nSi(R²)₄₋ₙ

wherein, R¹ and R² are each independently an alkyl group having 1-8 carbon atom(s); n is an integer of 1-4; when n is 1 or 2, then a plural of R² may be the same or different from each other; when n is 3 or 4,then a plural of R¹ may be the same or different from each other. Two or more above mentioned resins may be used together. In that case, a silicone resin having a silanol group which is contained in a layer of the water contact angle 3-8 degrees may be the same as the resin contained in the coating liquid or different from each other.

A silicone resin contained in the coating liquid may be a commercial product, named Fressera(R) (Panasonic Electric Co.) or the like.

An organic solvent contained in the coating liquid includes, but is not particularly limited to, for example an aliphatic hydrocarbon, an aromatic hydrocarbon, a chlorinated hydrocarbon, an ether type solvent, an alcohol type solvent of an aliphatic alcohol having carbon number 1-4 and 1-4 hydroxy group, a cellsolve type solvent such as ethylcellsolve, butylcellsolve, dioxane, methylacetate, dimethylformamide and the like. Two or more solvents may be used together.

A concentration of silicone resin in a coating liquid is preferably 0.001-1 weight%, and more preferably 0.1-1 weight%. When the concentration of silicone resin is less than 0.001 weight%, a uniform layer may not be formed. When the concentration of silicone resin is more than 20 weight%, the coating ability may be reduced.

A method for coating includes, but is not particularly limited to, for example a dipping coating, a spray coating, a spin casting or the like.

A substrate material includes, but is not particularly limited to, for example an organic material such as polycarbonate, PET (polyethyleneterephthalate), polystyrene, acrylics, and an in organic material such as gold, titanium, aluminium, iron, cupper, stainless, alumina, titanium oxide, zinc oxide and the like.

In the present invention, a molecular weight of a surface modifier is preferably 255-549 and more preferably 255-283. This enables an introduction of a phosphorylcholine-like group on the substrate surface at high density which may lead to reduce adsorption of a biological material.

A functional group having reactivity to the functional group of the surface modifier is not particularly limited. Specifically, a functional group having reactivity to amino group or hydroxyl group includes for example a carboxyl group, an aldehyde group or the like. A carboxyl group is preferable due to its high reactivity. Also, a functional group having reactivity to an aldehyde group or a carboxyl group includes for example amino group, hydroxyl group or the like. An amino group is preferable due to its high reactivity. Moreover, a functional group having reactivity to silanol group or a functional group capable of being hydrolyzed to form a silanol group includes a silanol group or a functional group which can be hydrolyzed to form a silanol group.

Furthermore a reactive functional group of a surface modifier is preferably bonded to a phosphorylcholine-like group via a spacer. A spacer includes, but is not particularly limited to, for example a methylene group, an oxyethylene group, an alkylene having at least one amino group or the like.

A surface modifier will be described hereinafter in detail.

### (a surface modifier having an amino group)

A surface modifier having an amino group includes, but is not particularly limited to, for example a compound disclosed in Japanese Patent Application Publication 2006-7203 and 2006-7204. Among the compound, a compound represented in a general formula 15 is preferable. 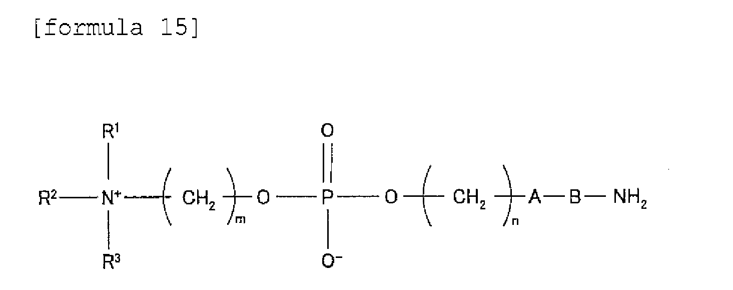 Wherein, R¹, R² and R³ is each independently an alkyl group having 1-6 carbon atom(s); A is an imino group, an ester bonding or an amide bonding; B is an alkylene having 1-3 carbon atom(s), a polyoxyethylene group having 1-3 carbon atom(s) or an arylene group; m is a integer of 2-6; n is 1 or 2.

The compound of the formula 15 having an imino group as A for example may be obtained as follows. Glycerophosphorylcholine is oxidized with periodic acid to produce a phosphorylcholine derivative having an aldehyde group. Then the product is reacted with a compound having an amino group for condensation reaction. Also, the compound of the formula 15 having an amide or ester bonding as A may be obtained as follows.

Glycerophosphorylcholine is oxidized with periodic acid and ruthenium trichloride to produce the phosphorylcholine derivative having a carboxyl group. Then the product is reacted with a compound having an amino group or a hydroxyl group for condensation reaction.

The compound of the formula 15 having an amide or an ester bonding as A may be obtained as follows. Glycerophosphorylcholine is oxidized with permanganic acid and hydrochloric acid to produce the phosphorylcholine derivative having a carboxyl group. Then the product is reacted with a compound having an amino group or a hydroxyl group for condensation reaction.

A method for producing a compound represented in the general formula 16 is described below specifically.

### (Example for producing a surface modifier A)

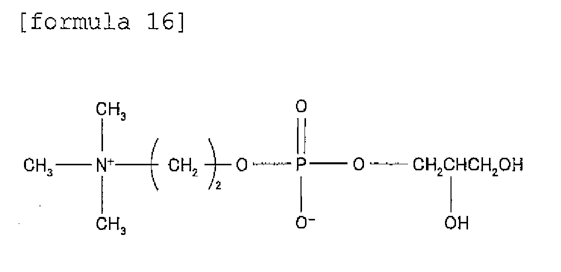

L-α-Glycerophosphorylcholine (commercially available) as represented by the formula 16 is dissolved in distilled water. After cooling the solution in an ice-water bath, sodium periodate is added into the solution under 5 hours stirring. The solvent is removed under vacuum and the residue is dried under vacuum. Methanol extraction of the residue gives a phosphorylcholine derivative shown in the structural formula 17.

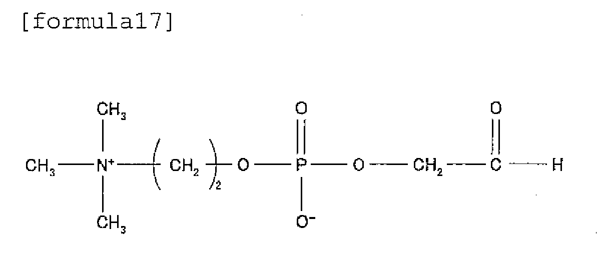

Then the phosphorylcholine derivative of the formula 17 is dissolved in methanol. An ethylenediamine was added to the methanol solution under stirring at room temperature. After cooling the mixture in an ice-water bath, sodium cyanotrihydroborate is added. Then the temperature of the mixture is back to the room temperature, and the mixture is stirred for 16 hours. All procedures are carried out under dry nitrogen stream. After removal of formed precipitates by filtration, the solution is condensed and dried under vacuum to give a compound A represented by the structural formula 18.

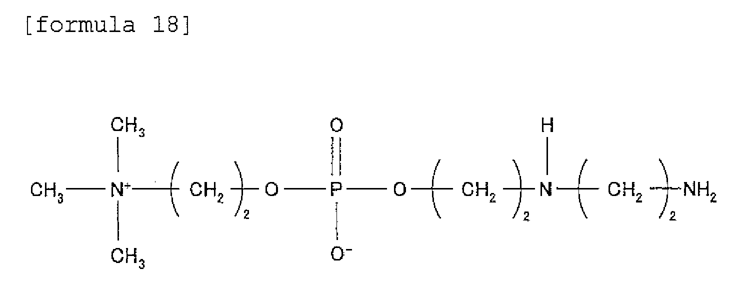

### (Example 1 for producing a surface modifier B)

Z-α-Glycerophosphorylcholine (commercially available) as represented by the formula 16 is dissolved in distilledwater. After cooling the solution in an ice-water bath, sodium periodate and ruthenium trichloride are added into the solution under 3 hours stirring. Then, methanol is added to the solution under further 30 min. stirring. After removal of formed precipitates by filtration, the solution is condensed and dried under vacuum to give the phosphorylcholine derivative represented by the structural formula 19.

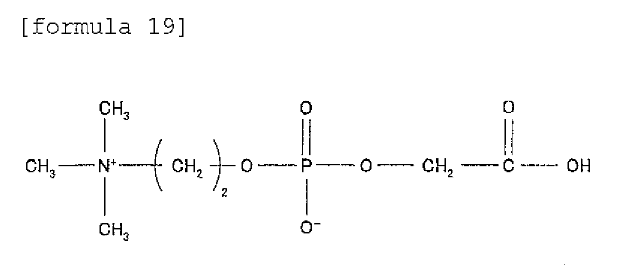

To the methanol solution of the phosphorylcholine derivative of the formula 19 is added ethylenediamine. After a triazine type dehydrocondensation agent (DMT-MM) is added to the solution, the mixture is stirred for 3 hours. After removal of formed precipitates by filtration, the solution is condensed and dried under vacuum to give a surface modifier B represented by the structural formula 20.

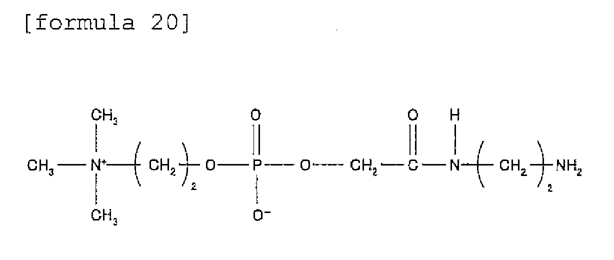

### (Example 2 for producing a surface modifier B)

L-α-Glycerophasphorylcholine (commercially available) as represented by the formula 16 is dissolved in hydrochloric acid under cooling in an ice-water bath. To the solution is added potassium permanganate and the mixture is stirred for 3 hours. Then methanol is added to the mixture, and the mixture is further stirred for 30 min. After removal of formed precipitates by filtration, the solution is condensed and dried under vacuum to give the phosphorylcholine derivative represented by the formula 19.

To the methanol solution of the phosphorylcholine derivative of the formula 19 is added an ethylenediamine. After a triazine type dehydrocondensation agent (DMT-MM) is added to the solution, the mixture is stirred for 3 hours. After removal of formed precipitates by filtration, the solution is condensed and dried under vacuum to give a surface modifier B represented by the structural formula 20.

An amide bond is formed by a reaction of a surface modifier having an amino group with a substrate having a carboxyl group on the surface. Specifically, after immersing the substrate in the solution of N-hydroxysuccinimide and 1-ethyl-3-(3-dimethylaminopropyl)carbodiimide to form an active ester group from a carboxyl group on the substrate, a surface modifier is added.

An imino bond is formed by a reaction of a surface modifier having amino group with a substrate having an aldehyde group on the surface. Specifically after 6 hours of keeping a substrate and a surface modifier in methanol at room temperature, sodium cyanotrihydroborate is added to the solution at 0 °C, and the mixutre is stirred overnight under heating. Other than methanol, a protic solvent such as water, ethanol, 2-propanol and the like may be used as a reaction solvent. It is preferable to use methanol as the solvent, since it has a tendency to give a higher introducing rate.

### (A surface modifier having a hydroxyl group)

A surface modifier having a hydroxyl group includes, but is not particularly limited to, for example L-α-Glycerophosphorylcholine or the like. A method for producing a surface modifier having a hydroxyl group is for example to reduce a phosphorylcholine derivative of formula 17 or phosphorylcholine derivative of formula 19 with for example sodium boronhydride.

An ester bond may be formed by a general condensation reaction of a surface modifier having a hydroxyl group with a substrate having a carboxyl group on the surface. Specifically, after activation of a hydroxyl group of the surface modifier by using bromocyan, a substrate is immersed.

An acetal bond may be formed by a general additional reaction of a surface modifier having a hydroxyl group with a substrate having an aldehyde group on the surface. Specifically, after keeping a substrate and a surface modifier in methanol for 6 hours at room temperature, sodium cyanohydroborate is added into the solution at 0 °C. The mixture is stirred overnight under heating. Other than methanol, a protic solvent such as water, ethanol, 2-propanol and the like may be used as a reaction solvent. It is preferable to use methanol as the solvent, since it has a tendency to give a higher introducing rate.

### (A surface modifier having an aldehyde group)

A surface modifier having an aldehyde group includes, but is not particularly limited to, for example, a compound disclosed in Japanese Patent Application Publication 2006-11383.

An acetal bond may be formed by a general additional reaction of a surface modifier having an aldehyde group with a substrate having a hydroxyl group on the surface. Specifically, after keeping a surface modifier and a substrate in methanol for 6 hours, sodium cyanohydroborate is added into the solution at 0°C. The mixture is stirred overnight under heating. Other than methanol, a protic solvent such as water, ethanol, 2-propanol and the like may be used as a reaction solvent. It is preferable to use methanol as the solvent, since it has a tendency to give a higher introducing rate.

An imino bond may be formed by a general condensation reaction of a surface modifier having an aldehyde group with a substrate having an amino group on the surface. Specifically, after keeping a surface modifier and a substrate in methanol for 6 hours, sodium cyanohydroborate is added into the solution at 0°C. The mixture is stirred overnight under heating. Other than methanol, a protic solvent such as water, ethanol, 2-propanol and the like may be used as a reaction solvent. It is preferable to use methanol as the solvent, since it has a tendency to give a higher introducing rate.

### (A surface modifier having an carboxyl group)

A surface modifier having a carboxyl group includes, but is not particularly limited to, for example a compound disclosed in Japanese Patent Application Publication 2006-11381.

An ester bond may be formed by a general condensation reaction of a surface modifier having a carboxyl group with a substrate having a hydroxyl group on the surface. Specifically, after activation of the hydroxyl group of the surface modifier by using bromocyan, the substrate is immersed.

An amide bond may be formed by a general condensation reaction of a surface modifier having a carboxyl group with a substrate having an amino group on the surface. Specifically, after active esterification of carboxyl group of the surface modifier by using N-hydroxysuccinimide and
1-ethyl-3-(3-dimetylaminopropyl)carbodiimide, the substrate is immersed.

### (A surface modifier having a silanol group and/or a functional group capable of being hydrolyzed to form silanol group)

A functional group capable of being hydrolyzed to form a silanol group includes, for example hydrosilyl group, alkoxysilyl group, halosilyl group, acyloxysilyl group, aminosilyl group or the like. An alkoxysilyl group having 1-6 carbon atom (s) or a hydrosilyl group is preferable from stability or reactivity point of view. A surface modifier having a silanol group and/or a functional group capable of being hydrolyzed to form a silanol group includes, but is not particularly limited to, a compound disclosed in Japanese Patent Application Publication 2006-11380.

A silanol group may be formed by coating a coating liquid containing a surface modifier having a silanol group and/or a functional group capable of being hydrolyzed to form silanol group on a substrate having a silanol group and/or a functional group capable of being hydrolyzed to form a silanol group, optionally followed by hydrolysis of a functional group capable of being hydrolyzed to form silanol group. Consequently the surface of the substrate may be improved by a dehydrocondensation between a silanol group from the surface modifier and a silanol group on the substrate. Specifically, when a functional group capable of being hydrolyzed to form a silanol group is hydrolyzed, a substrate is coated by a coating liquid and then water, an acid or an alkaline may be coated or the substrate may be heated. Alternatively, a coating liquid may be coated on the substrate after coating water, an acid or an alkaline on the substrate. Further water, an acid or alkaline may be mixed with a coating liquid. In this case a coating liquid is preferably prepared timely on coating, since hydrolysis may be taking place in the coating liquid. In the case of using water, an acid or an alkaline, the reaction typically takes place sufficiently at room temperature, although heat may be applied. Also in the case of not using any water, an acid nor an alkaline, the reaction may take place gently under atmospheric moisture. An acid or an alkaline for hydrolysis reaction is not particularly limited but may be any compound capable of promoting a hydrolysis reaction. A mixture of two or more of those may be used. Also an acid or an alkaline for hydrolysis reaction may be used in aqueous solution.

A coating liquid may be a solution or a dispersion of a surface modifier in an organic solvent. The organic solvent includes, but is not particularly limited to, an aliphatic hydrocarbon, an aromatic hydrocarbon, a chlorinated hydrocarbon, an ether type solvent, an alcohol type solvent of an aliphatic alcohol having carbon number 1-4 and 1-4 hydroxyl group, a cellsolve type solvent such as ethylcellsolve, butylcellsolve and the like, dioxane, methyl acetate, dimethylformamide or the like. A mixture of solvent having 2 or more of those solvents may be used.

The concentration of a surface modifier is preferably 0.1-30 weight% and more preferably 1-10 weight%. When the concentration of a surface modifier is less than 0.1 weight%, a surface modifier may not be coated sufficiently by one time treatment. When the concentration of a surface modifier is more than 30 weight%, coating properties or the like may be reduced.

A coating method includes, but is not particularly limited to, a dipping coating, a spray coating, a spin casting or the like.

A biotip of the present invention may be produced by a reaction of a surface modified substrate of the present invention with a ligand having a functional group having reactivity to a reactive functional group on the surface of substrate. This may introduce the ligand and phosphorylcholine-like group to the surface of the substrate at high density. Accordingly, thus obtained biotip has superior trapping efficiency of a specified material, and also may prevent any adsorption of other biological material.

In the present invention a reactive functional group of a ligand to a functional group having reactivity is not particularly limited. Specifically a reactive functional group to an amino group or a hydroxyl group includes for example a carboxyl group, an aldehyde group or the like. A carboxyl group is preferable due to its high reactivity. Also a reactive functional group to an aldehyde group or a carboxyl group includes for example an amino group, a hydroxyl group or the like. An amino group is preferable due to its high reactivity. Further a reactive functional group to a silanol group or a functional group capable of being hydrolyzed to form a silanol group includes, for example, a silanol group or a functional group capable of being hydrolyzed to form a silanol group. A functional group capable of being hydrolyzed to form a silanol group includes, for example, a hydrosilyl group, an alkoxysilyl group, a halosilyl group, an acyloxysilyl group, an aminosilyl group or the like. An alkoxylsilyl group having 1-6 carbon atom(s) or a hydrosilyl group is preferable due to the stability, reactivity or the like.

A ligand and a reactive functional group are preferably bonded via a spacer. A spacer includes, but is not particularly limited to, for example a methylene group, an oxyethylene group, an alkylene group having at least one amino group or the like.

A ligand includes, but is not particularly limited to, for example an antibody such as IgG, IgM, IgA, IgD, IgE, IgY or the like; an antigen such as a protein or a polysaccharide; an enzyme such as a glutathione-S-transferase or the like; a substrate such as glutathione; a receptor such as hormone receptor, cytokine receptor or the like; a peptide, a DNA, a RNA, aptamer, a Protein A, a Protein G, an avidin, a biotin; a chelate compound such as a nitrilotriacetic acid; a metal ion such as Ni²⁺, Co²⁺, Cu²⁺, Zn²⁺, Fe³⁺ and the like.

In the case of a protein as a ligand, an imino bond may be formed by a general condensation reaction of an amino group of the protein with an aldehyde group on the surface of the substrate. Specifically after 6 hours keeping a substrate and a protein in methanol at room temperature, sodium cyanotrihydroborate is added to the solution at 0 °C, and the mixture is stirred overnight under heating. Other than methanol, a protic solvent such as water, ethanol, 2-propanol and the like may be used as a reaction solvent. It is preferable to use methanol as the solvent, since it has a tendency to give a higher introducing rate.

Also an amide bond is formed by a general condensation reaction of an amino group of a protein with a substrate having a carboxyl group on the surface. Specifically after immersing the substrate in the solution of N-hydroxysuccinimide and
1-ethyl-3-(3-dimethylaminopropyl)carbodiimide to form an active ester group from a carboxyl group on the substrate, a protein is added.

An imino bond is formed by a general condensation reaction of an amino group of a protein with a substrate having an amino group on the surface via a glutaraldehyde. Specifically, after a substrate (or a protein) is reacted with glutaraldehyde, the substrate (or a protein) is reacted with a protein (or a substrate).

Also, an ester bond may be formed by a general condensation reaction of a carboxyl group of a protein with a substrate having a hydroxyl group on the surface. Specifically, after activation of a hydroxyl group of the substrate by using bromocyan, a protein is added.

A functional group having a reactivity to a reactive functional group of a ligand may be the same as a functional group having a reactivity to a reactive functional group of a surface modifier or not the same.

A method for producing a biotip of the present invention comprises steps of reacting a substrate having a reactive functional group on the surface with a ligand having a functional group being reactive to the reactive functional group, and reacting the substrate reacted with the ligand with a compound represented by the general formula 21, 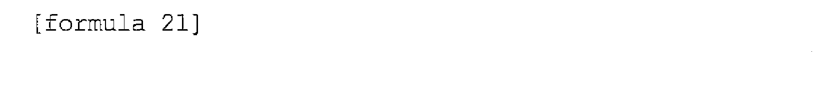 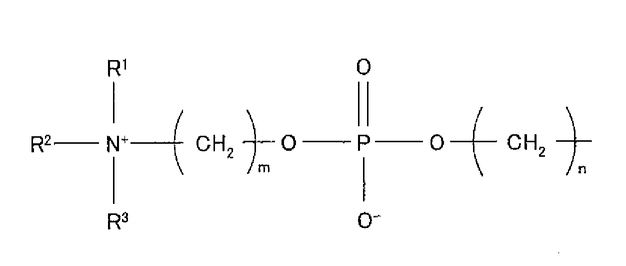 wherein, R¹, R² and R³ are each independently an alkyl group with a carbon number of 1 or more and 6 or less, m is an integer from 2 to 6, and n is 1 or 2.

Thus produced biotip may selectively trap an object material which may selectively bind to a ligand by immersing the biotip into a sample solution containing the subject material. A biotip includes, but is not particularly limited to, for example a protein tip, a DNA tip or the like.

Also, a biotip of the present invention may be applied for a biosensor such as a blood sugar sensor, a BOD sensor, a DNA sensor or the like. A blood sugar sensor may contain a sensor tip having a glucoseoxidase fixed on the surface, and may analyze the glucose concentration in the blood based on an electrochemical or a colorimetric analysis for hydrogen peroxide generated by glucose oxidation.

### EXAMPLE

### Example 1

After a 96-well plate made of polystyrene was washed with 2-propanol, the well was immersed in the mixture of 0.21 ml of 2-propanol and 0.09 ml of Fressera (Panasonic Electric Co), and the well then was dried at room temperature for 1 hour to introduce a silanol group on the surface of the well.

Then after the well was washed with methanol, the well was coated with a 0.2 ml methanol solution of 2 mg of 3- (trimethoxysilyl) propyl succinic anhydride, and dried 5 hours at room temperature, washed with water, and dried to introduce a carboxyl group on the surface of the well.

Then to the well was added a liquid prepared by adjusting pH at 4.5 with hydrochloric acid of the solution obtained by dissolving 3 mg of N-hydroxysuccinimide and 3 mg of 1-ethyl-3- (3-dimethylaminopropyl) carbodiimide in 0.3 ml of ultra pure water. The well was kept for 1 hour to change the carboxyl group to the active ester. Then to the well was added a 0.2 ml of phosphate buffer saline (PBS) dissolving 1 mg of an anti-human albumin antibody to introduce an anti-human albumin antibody on the surface of the well.

Then the well was washed with PBS. To the well was added a liquid prepared by adjusting pH at 7.5 with hydrochloric acid of the solution obtained by dissolving 6mg of surface modifier B in 0.2 ml of ultra pure water. The well was kept for 1 hour at room temperature to introduce a phosphorylcholine group on the surface of the well and thus produce a biotip.

### Comparative Example 1

A biotip was produced as Example 1, except introducing a hydroxyl group on the surface of the well by using an ethanolamine instead of a surface modifier B.

### [Signal Test]

To the well of Example 1 or Comparative Example 1 was added 0.1 ml of 1 mg/ml PBS solution of human albumin. Then the well was kept 1 hour at room temperature to complete the reaction. The well was washed with PBS. To the well was added 0.1 ml of 1 µg/ml PBS of HRP-labeled human albumin antibody. The well was kept 1 hour to complete the reaction. The well was washed with PBS. The well was treated with substrate TMBZ and analyzed colorimetrically by measuring an absorption at 450 nm wavelength. As shown in FIG. 1, both biotip of Example 1 and Comparative Example 1 gave the same degree of 450 nm absorption (signal). This result suggests that an amount of HRP-labeled human albumin antibody fixed via human albumin to an anti-human albumin antibody on the surface of the well is the same degree between both tips.

### [Noise Test]

To the biotip of Example 1 or Comparative Example 1 was added 1 ml of PBS. The well was kept 1hour at room temperature. The well was washed with PBS. To the well was added 0.1 ml of 1 µg/ml PBS of HRP-labeled human antibody. The well was kept 1 hour to complete the reaction. The well was washed with PBS. The well was treated with substrate TMBZ and analyzed colorimetrically by measuring an absorption at 450 nm wavelength. As a result, the biotip of Example 1 has a noise only about a half of the noise of the biotip of Comparative Example 1 (FIG 1). This suggests that an amount of HRP-labeled human albumin antibody fixed on the surface of the well of the Example 1 is about a half of the amount of Comparative Example 1. Accordingly it may be concluded that a biotip of Example 1 is highly effective to reduce protein absorption, since a phosphorylcholine group is introduced on the surface of the well at high density.

These results indicate that a biotip of Example 1 has a better S/N ratio than a biotip of Comparative Example 1.

The present international application claims the priority based on Japanese Patent Application No.2009-47518 filed on March 2, 2009, and the entire content of Japanese Patent Application No.2009-47518 is incorporated by reference in the present international application.

## Claims

1. A surface modified substrate produced by a method, the method comprising:
reacting a substrate having a reactive functional group on a surface thereof with a functional group being reactive to the reactive functional group; and
reacting the substrate with a compound represented by the general formula, 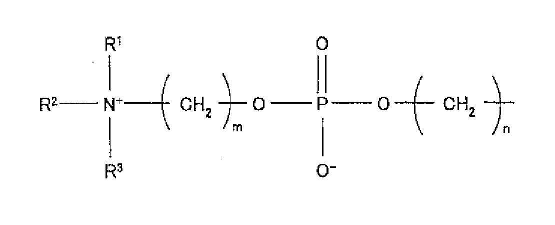
wherein R¹, R² and R³ are each independently an alkyl group with a carbon number of 1 or more and 6 or less, m is an integer from 2 to 6, and n is 1 or 2.

2. The surface modified substrate as claimed in claim 1,
wherein the reactive functional group is one or more groups selected from a group consisting of an amino group, a hydroxyl group, an aldehyde group, a carboxyl group, a functional group which may be hydrolyzed to produce a silanol group, and a silanol group.

3. A biotip produced by a method comprising reacting the surface modified substrate according to claim 1 with a ligand having a functional group being reactive to the reactive functional group.

4. A method for producing a biotip comprising steps of:
reacting a substrate having a reactive functional group on a surface thereof with a ligand having a functional group being reactive to the reactive functional group; and
reacting the substrate reacted with the ligand with a compound represented by the general formula, 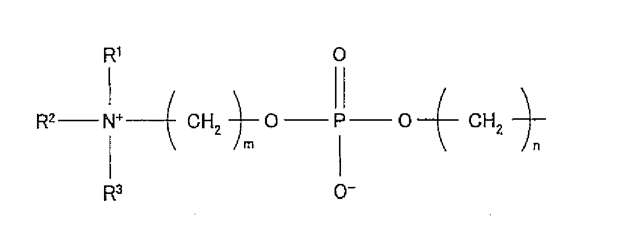
wherein R¹, R² and R³ are each independently an alkyl group with a carbon number of 1 or more and 6 or less, m is an integer from 2 to 6, and n is 1 or 2.

5. A biotip produced by the method for producing a biotip according to claim 4.

6. A biosensor comprising the biotip according to claim 3 or 5.

7. An analytical method for a material being selectively bound to a ligand, comprising using the biosensor according to claim 6.
